# EUROPEAN PATENT SPECIFICATION

(11) **EP 2 393 800 B1**
(45) Date of publication and mention of the grant of the patent: **08.10.2014**
(21) Application number: 10703768.1
(22) Date of filing: 04.02.2010
(51) Int. Cl.: C07D 401/06

(54) **PYRIDINE COMPOUNDS AS SUBTYPE SELECTIVE MODULATORS OF ALPHA2B AND/OR ALPHA 2C ADRENERGIC RECEPTORS**
PYRIDINVERBINDUNGEN ALS UNTERTYPSELEKTIVE MODULATOREN VON ALPHA2B- UND/ODER ALPHA2C-ADRENERGEN REZEPTOREN
COMPOSÉS PYRIDINES EN TANT QUE MODULATEURS SÉLECTIFS DE SOUS-TYPE DE RÉCEPTEURS ADRÉNERGIQUES ALPHA 2B ET/OU ALPHA 2C

(30) Priority: 06.02.2009 US 150448 P; 13.02.2009 US 152398 P
(43) Date of publication of application: 14.12.2011
(73) Proprietor: ALLERGAN, INC., Irvine, CA 92612 (US)
(72) Inventor: NGUYEN, Phong X., Placentia, California 92870 (US); HEIDELBAUGH, Todd M., Fountain Valley, California 92708 (US); SINHA, Santosh C., Ladera Ranch, California 92694 (US); CHOW, Ken, Newport Coast, California 92657 (US); GARST, Michael E., Newport Beach, California 92660 (US); GIL, Daniel W., Corona Del Mar, California 92625 (US)
(74) Representative: HOFFMANN EITLE
(86) International application number: PCT/US2010/023241
(87) International publication number: WO 2010/091209

(56) References cited:
- US-A- 5 866 579
- US-A1- 2009 176 843
- YOSHIYA AMEMIYA ET AL: "Synthesis and alpha-adrenergic activities of 2- and 4-substituted imidazoline and imidazole analogues" JOURNAL OF MEDICINAL CHEMISTRY, AMERICAN CHEMICAL SOCIETY, WASHINGTON, US LNKD- DOI:10.1021/JM00082A017, vol. 35, no. 4, 1 January 1992 (1992-01-01), pages 750-755, XP002151512 ISSN: 0022-2623

## Description

### Cross-Reference

This application claims the benefit of U.S. Provisional Patent Application Serial Number 61/150,448, filed February 6, 2009 and U.S. Provisional Patent Application Serial Number 61/152,398, filed on February 13, 2009, which the entire disclosure of both are incorporated herein by this specific reference.

### Field of the Invention

The present invention relates generally to compounds and methods for treating disorders associated with selective subtype modulation of alpha 2B and alpha 2C adrenergic receptors. The invention relates more specifically to the use of certain 4-[1-(1H-Imidazol-4-yl)-ethyl]-2,3-substituted pyridine compounds and pharmaceutical compositions thereof to treat disorders associated with selective subtype alpha 2B and 2C adrenergic receptor modulation.

### Background

Human adrenergic receptors are integral membrane proteins which have been classified into two broad classes, the alpha and the beta adrenergic receptors. Both types mediate the action of the peripheral sympathetic nervous system upon binding of catecholamines, norepinephrine and epinephrine.

Norepinephrine is produced by adrenergic nerve endings, while epinephrine is produced by the adrenal medulla. The binding affinity of adrenergic receptors for these compounds forms one basis of the classification: alpha receptors tend to bind norepinephrine more strongly than epinephrine and much more strongly than the synthetic compound isoproterenol. The preferred binding affinity of these hormones is reversed for the beta receptors. In many tissues, the functional responses, such as smooth muscle contraction, induced by alpha receptor activation are opposed to responses induced by beta receptor binding.

Subsequently, the functional distinction between alpha and beta receptors was further highlighted and refined by the pharmacological characterization of these receptors from various animal and tissue sources. As a result, alpha and beta adrenergic receptors were further subdivided into α₁, α₂, β₁, and β₂ subtypes. Functional differences between α₁ and α₂ receptors have been recognized, and compounds which exhibit selective binding between these two subtypes have been developed. Thus, in published international patent application WO 92/0073, the selective ability of the R(+) enantiomer of terazosin to selectively bind to adrenergic receptors of the α₁ subtype was reported. The α₁/α₂ selectivity of this compound was disclosed as being significant because agonist stimulation of the α₂ receptors was said to inhibit secretion of epinephrine and norepinephrine, while antagonism of the α₂ receptor was said to increase secretion of these hormones. Thus, the use of non-selective alpha-adrenergic blockers, such as phenoxybenzamine and phentolamine, was said to be limited by their α₂ adrenergic receptor mediated induction of increased plasma catecholamine concentration and the attendant physiological sequelae (increased heart rate and smooth muscle contraction). For a further general background on the α-adrenergic receptors, the reader's attention is directed to Robert R. Ruffolo, Jr., α-Adrenoreceptors: Molecular Biology, Biochemistry and Pharmacology, (Progress in Basic and Clinical Pharmacology series, Karger, 1991), wherein the basis of α₁/α₂ subclassification, the molecular biology, signal transduction, agonist structure-activity relationships, receptor functions, and therapeutic applications for compounds exhibiting α-adrenergic receptor affinity is explored.

The cloning, sequencing and expression of alpha receptor subtypes from animal tissues has led to the subclassification of the α₁ adrenoreceptors into α_{1A}, α_{1B}, and α_{1D}. Similarly, the α₂ adrenoreceptors have also been classified α_{2A}, α_{2B}, and α_{2C} receptors. Each α₂ receptor subtype appears to exhibit its own pharmacological and tissue specificities. Compounds having a degree of specificity for one or more of these subtypes may be more specific therapeutic agents for a given indication than an α₂ receptor pan-agonist (such as the drug clonidine) or a pan-antagonist.

Among other indications, such as the treatment of glaucoma, hypertension, sexual dysfunction, and depression, certain compounds having alpha₂ adrenergic receptor agonist activity are known analgesics. However, many compounds having such activity do not provide the activity and specificity desirable when treating disorders modulated by alpha₂ adrenoreceptors. For example, many compounds found to be effective agents in the treatment of pain are frequently found to have undesirable side effects, such as causing hypotension and sedation at systemically effective doses. There is a need for new drugs that provide relief from pain without causing these undesirable side effects. Additionally, there is a need for agents which display activity against pain, particularly chronic pain, such as chronic neuropathic and visceral pain.

### Detailed Description of the Invention

The compound of the present invention is as follows: 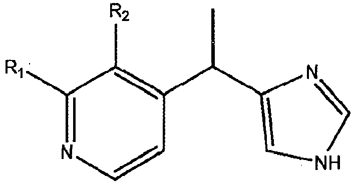 Wherein:
**R₁** and **R₂** independently= H, H₂OR₃, C₁-C₄ alkyl, halogens, CF₃,
**R₃** = H, C₁ - C₄ alkyl
**R₁** and **R₂** may be fused to form a saturated ring

   -CH₂-X-(CH₂)ₙ-CH₂-
X-CH₂,
n = 0,1,2
and any combination thereof or pharmaceutically acceptable salts, tautomers, enantiomers and diastereomers.

Unless otherwise indicated, reference to a compound should be construed broadly to include pharmaceutically acceptable salts, prodrugs, tautomers, alternate solid forms, and non-covalent complexes of a chemical entity of the depicted structure or chemical name.

A pharmaceutically acceptable salt is any salt that retains the activity of the parent compound and does not additional unacceptable deleterious or untoward effects on the subject to which it is administered and in the context in which it is administered compared to the parent compound. A pharmaceutically acceptable salt also refers to any salt which may form in vivo as a result of administration of an acid, another salt, or a prodrug which is converted into an acid or salt.

Pharmaceutically acceptable salts of acidic functional groups may be derived from organic or inorganic bases. The salt may comprise a mono or polyvalent ion. Of particular interest are the inorganic ions, lithium, sodium, potassium, calcium, and magnesium. Organic salts may be made with amines, particularly ammonium salts such as mono-, di- and trialkyl amines or ethanol amines. Salts may also be formed with caffeine, tromethamine and similar molecules. Hydrochloric acid or some other pharmaceutically acceptable acid may form a salt with a compound that includes a basic group, such as an amine or a pyridine ring.

A prodrug is a compound which is converted to a therapeutically active compound after administration. While not intending to limit the scope of the invention, conversion may occur by hydrolysis of an ester group or some other biologically labile group. Generally, but not necessarily, a prodrug is inactive or less active than the therapeutically active compound to which it is converted. Ester prodrugs of the compounds disclosed herein are specifically contemplated. An ester may be derived from a carboxylic acid of C1 (i.e. the terminal carboxylic acid of a natural prostaglandin), or an ester may be derived from a carboxylic acid functional group on another part of the molecule, such as on a phenyl ring. While not intending to be limiting, an ester may be an alkyl ester, an aryl ester, or a heteroaryl ester. The term alkyl has the meaning generally understood by those skilled in the art and refers to linear, branched, or cyclic alkyl moieties. C₁₋₆ alkyl esters are particularly useful, where alkyl part of the ester has from 1 to 6 carbon atoms and includes, but is not limited to, methyl, ethyl, propyl, isopropyl, *n*-butyl, *sec*-butyl, *iso-*butyl, *t*-butyl, pentyl isomers, hexyl isomers, cyclopropyl, cyclobutyl, cyclopentyl, cyclohexyl, and combinations thereof having from 1-6 carbon atoms, etc.

Tautomers are isomers that are in rapid equilibrium with one another. They often, but do not necessarily, include a transfer of a proton, hydrogen atom, or hydride ion. For example, the structures herein are intended to include, but are not limited to, the tautomeric forms shown below.

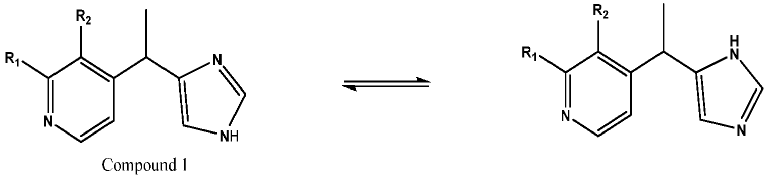

Unless stereochemistry is explicitly depicted, a structure is intended to include every possible stereoisomer, both pure or in any possible mixture.

Alternate solid forms are different solid forms than ones that may result from practicing the procedures described herein. For example, alternate solid forms may be polymorphs, different kinds of amorphous solid forms, glasses, and the like.

Non-covalent complexes are complexes that may form between the compound and one or more additional chemical species that do not involve a covalent bonding interaction between the compound and the additional chemical species. They may or may not have a specific ratio between the compound and the additional chemical species. Examples might include solvates, hydrates, charge transfer complexes, and the like.

Compounds of structures such as those shown below are contemplated: 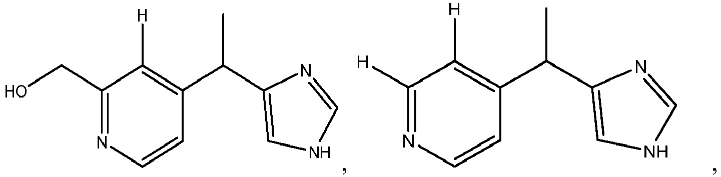 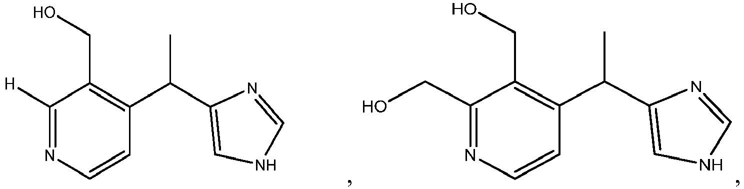 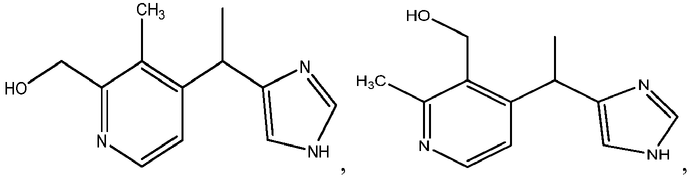 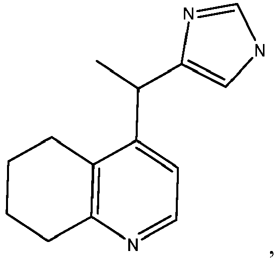 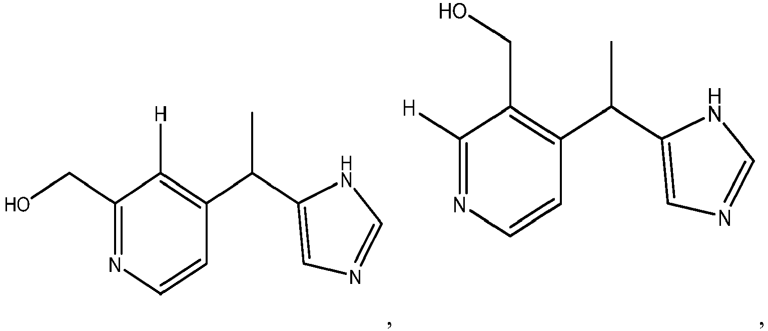 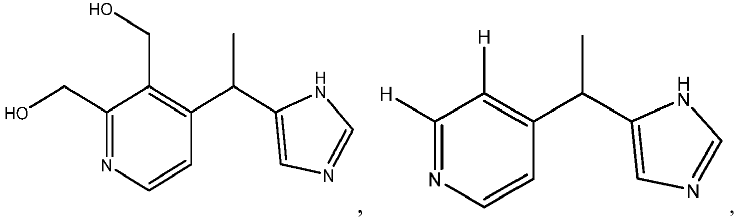 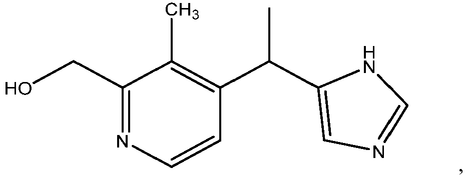 and,

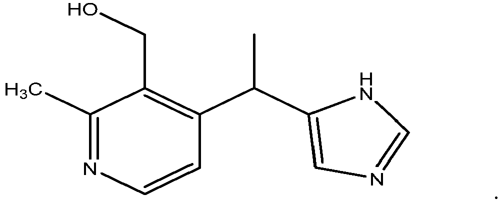

The compounds will be useful for the treatment of mammals, including humans, with a range of conditions and diseases that are alleviated by alpha 2B, 2C activation, including but not limited to treating ischemic neuropathies, optic neuropathy, pain, visceral pain, corneal pain, headache pain, migraine, cancer pain, back pain, irritable bowel syndrome pain, muscle pain and pain associated with diabetic neuropathy, the treatment of diabetic retinopathy, other retinal degenerative conditions, stroke, cognitive deficits, neuropsychiatric conditions, drug dependence and addiction, withdrawal symptoms, obsessive-compulsive disorders, obesity, insulin resistance, stress-related conditions, diarrhea, diuresis, nasal congestion, spasticity, attention deficit disorder, psychoses, anxiety, depression, autoimmune disease, Crohn's disease, gastritis, Alzheimer's, Parkinson's ALS, and other neurodegenerative diseases.

Alpha-2 adrenergic receptors have been characterized by molecular and pharmacological methods which include alpha 1A, alpha 1B , alpha 1D, alpha 2A, alpha 2B and alpha 2C. Activation of these alpha receptors evokes physiological responses. Adrenergic modulator compounds described in this invention activate one or both of the alpha 2B and alpha 2C receptors and resulting in useful therapeutic actions.

The compounds were synthesized and tested for alpha adrenergic activity using the Receptor Selection and Amplification Technology (RSAT) assay (Messier et. al., 1995, Pharmacol. Toxicol. 76, pp. 308-311). Cells expressing each of the alpha-2 adrenergic receptors alone were incubated with the Compound 1 and a receptor-mediated growth response was measured. A compound's activity is expressed as its relative efficacy compared to a standard full agonist (see table). Compound of this invention activates alpha-2B and alpha-2C receptors.

It is known that chronic pain (such as pain from cancer, arthritis, and many neuropathic injuries) and acute pain (such as that pain produced by an immediate mechanical stimulus, such as tissue section, pinch, prick, or crush) are distinct neurological phenomena mediated to a large degree either by different nerve fibers and neuroreceptors or by a rearrangement or alteration of the function of these nerves upon chronic stimulation. Sensation of acute pain is transmitted quite quickly, primarily by afferent nerve fibers termed C fibers, which normally have a high threshold for mechanical, thermal, and chemical stimulation. While the mechanisms of chronic pain are not completely understood, acute tissue injury can give rise within minutes or hours after the initial stimulation to secondary symptoms, including a regional reduction in the magnitude of the stimulus necessary to elicit a pain response. This phenomenon, which typically occurs in a region emanating from (but larger than) the site of the original stimulus, is termed hyperalgesia. The secondary response can give rise to profoundly enhanced sensitivity to mechanical or thermal stimulus.

The A afferent fibers can be stimulated at a lower threshold than C fibers, and appear to be involved in the sensation of chronic pain. For example, under normal conditions, low threshold stimulation of these fibers (such as a light brush or tickling) is not painful. However, under certain conditions such as those following nerve injury or in the herpes virus-mediated condition known as shingles the application of even such a light touch or the brush of clothing can be very painful. This condition is termed allodynia and appears to be mediated at least in part by A afferent nerves. C fibers may also be involved in the sensation of chronic pain, but if so it appears clear that persistent firing of the neurons over time brings about some sort of change which now results in the sensation of chronic pain.

By "acute pain" is meant immediate, usually high threshold, pain brought about by injury such as a cut, crush, burn, or by chemical stimulation such as that experienced upon exposure to capsaicin, the active ingredient in chili peppers.

By "chronic pain" is meant pain other than acute pain, such as, without limitation, neuropathic pain, visceral pain (including that brought about by Crohn's disease and irritable bowel syndrome (IBS)), and referred pain.

For the purposes of this disclosure, "treat," "treating," or "treatment" refer to the use of a compound, composition, therapeutically active agent, or drug in the diagnosis, cure, mitigation, treatment, prevention of disease or other undesirable condition.

These compounds may be formulated into solid, liquid, or other types of dosage forms using methods known in the art. Both formulation of dosage forms and determination of a therapeutically effective dose can be readily made by a person of ordinary skill using routine methods.

### GENERAL METHODS FOR OBTAINING THE COMPOUNDS

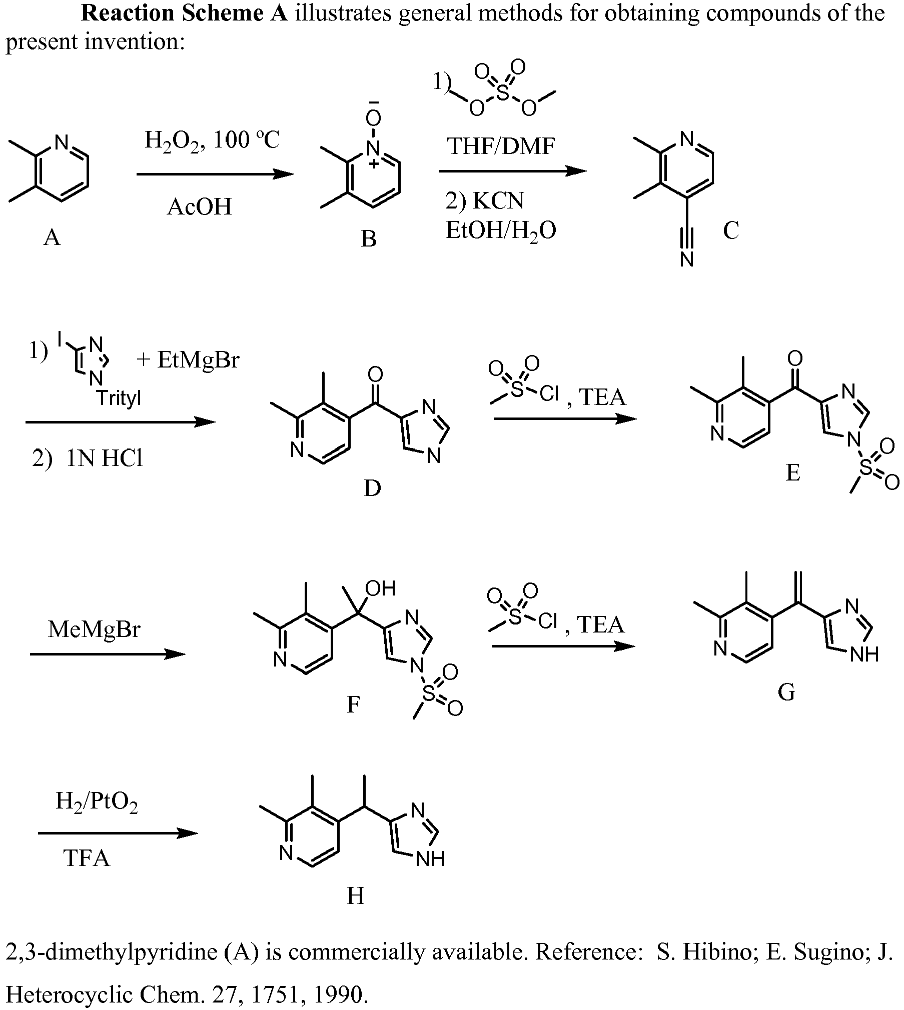

2,3-dimethylpyridine 1-oxide (B): A solution of A (5.00 ml, 0.0436 mol) and hydrogen peroxide (15% in water, 7.5 ml) in acetic acid (50.0 ml) was heated at 100 degree Celsius for 3.5 hours. TLC analysis showed some A remained. Hydrogen peroxide (30% in water, 3 ml) was added to the reaction solution, which was kept at 100 degree Celsius for another 10 hours. The solution was cooled to room temperature. The acetic acid was removed, and the residue was basified with sodium carbonate (aq) to pH around 7. The aqueous layer was extracted with a solution of chloroform/isopropanol (3:1) until all the UV active material was in the organic layers. The pooled organic layers were dried over magnesium sulfate, filtered, and concentrated under vacuum. The crude product was purified by chromatography on silica gel (100% ethyl acetate, then 3-4% ammonia methanol in dichloromethane) to give 2,3-dimethylpyridine 1-oxide (B) as a white solid.

2,3-dimethylisonicotinonitrile (C): A solution of (B) (4.38 g, 0.0355 mol) in THF/DMF (9:1) was added methyl sulfate (3.70 ml, 0.0388 mol). The solution was stirred at room temperature over night. A solution of potassium cyanide (2.84 g, 0.0423 mol) in EtOH/water (1:1, 66 ml) was added to the previous solution, and the resulting one was stirred at room temperature over night. THF was removed. The residue was added water, and extracted with chloroform. The pooled organic layers were washed with water twice, dried over magnesium sulfate, and filtered. The filtrate was added silica gel, and solvent was removed. Purification by chromatography on silica gel (20% ethyl acetate, 80% hexane) gave 2,3-dimethylisonicotinonitrile (C).

(2,3-dimethylpyridin-4-yl)(1H-imidazol-4-yl)methanone (D): A solution of 4-iodo-1-trityl-1H-imidazole (5.20 g, 0.0119 mol) in anhydrous dichloromethane (50.0 mL) was added ethyl magnesium bromide (3.0 M in diethyl ether, 4.00 ml, 0.0120 mol) drop wise. The reaction mixture was stirred at room temperature for one hour. C (0.780 g, 0.00590 mol) in dichloromethane was added to the reaction mixture via addition funnel. The reaction mixture was stirred at room temperature over night, and quenched with ammonium chloride (sat.) The aqueous layer was extracted with dichloromethane. The pooled organic layers were washed with brine, dried over magnesium sulfate, and filtered. Dichloromethane was removed, and the residue in THF/MeOH was added HCl (1N). The resulting mixture was stirred at room temperature over night. THF was removed, and the aqueous layer was basified with NaOH (2N) to pH above 7. The aqueous layer was extracted with chloroform/isopropanol (3:1). The pooled organic layers were dried over magnesium sulfate, and filtered. The filtrate was added silica gel, and solvents were removed under vacuum. Purification by chromatography on silica gel (4-5% NH3 MeOH in dichloromethane) gave crude (2,3-dimethylpyridin-4-yl)(1H-imidazol-4-yl)methanone (D) as a white solid, which was used in the next step.

(2,3-dimethylpyridin-4-yl)(1-(methylsulfonyl)-1H-imidazol-4-yl)methanone (E): D (0.590 g, 0.00293 mol) in dioxane was added triethyl amine (3.30 ml, 0.0236 mol), and cooled to 0 degree Celsius. Methanesulfonyl chloride (0.570 ml, 0.00736 mol) was added, and the resulting mixture was stirred for 5.5 hours. TLC analysis showed some starting material remained. Methanesulfonyl chloride (0.30 ml, 0.00388 mol) was added, and the reaction mixture was stirred at room temperature over night. Solvents were removed under vacuum. The residue was added water, and extracted with dichloromethane. The pooled organic layers were dried over magnesium sulfate, and filtered. The filtrate was added silica gel, and the solvents were removed under vacuum. Purification by chromatography on silica gel (2% NH3 MeOH in dichloromethane) gave (2,3-dimethylpyridin-4-yl)(1-(methylsulfonyl)-1H-imidazol-4-yl)methanone (E) as a white solid.

1-(2,3-dimethylpyridin-4-yl)-1-(1-(methylsulfonyl)-1H-imidazol-4-yl)ethanol (F): A solution of E (0.560 g, 0.00201 mol) in THF at 0 degree Celsius was added methyl magnesium bromide (3.0 M in diethyl ether, 1.40 ml, 0.0042 mol). The resulting mixture was stirred over night. THF was removed. The residue was added ammonium chloride (aq), and extracted with chloroform/isopropanol (3:1). The pooled organic layers were dried over magnesium sulfate, and filtered. The filtrate was added silica gel, and the solvents were removed. Purification by chromatography on silica gel (3-5% NH3 MeOH in dichloromethane) gave 1-(2,3-dimethylpyridin-4-yl)-1-(1-(methylsulfonyl)-1H-imidazol-4-yl)ethanol (F).

4-(1-(1H-imidazol-4-yl)vinyl)-2,3-dimethylpyridine (G): A solution of F (0.300 g, 0.00102 mol) in dichloromethane was added triethyl amine (1.15 ml, 0.00825 mol). The resulting mixture was cooled to 0 degree Celsius, and added methanesulfonyl chloride (0.240 ml, 0.00310 mol). The reaction mixture was stirred at room temperature over night. The reaction mixture was quenched with water, and extracted with chloroform/isopropanol (3:1). The pooled organic layers were dried over magnesium sulfate, and filtered. The filtrate was added silica gel, and the solvents were removed under vacuum. Purification by chromatography on silica gel (3% NH3 MeOH in dichloromethane) gave 4-(1-(1H-imidazol-4-yl)vinyl)-2,3-dimethylpyridine (G) as a white solid.

4-(1-(1H-imidazol-4-yl)ethyl)-2,3-dimethylpyridine (H): G (0.0700 g, 0.000352 mol) in TFA was added platinum oxide (40.0 mg). The mixture was hydrogenated at 50 psi for 3 hours. The mixture was filtered to remove platinum oxide. TFA was removed under vacuum. The residue was basified with NaOH (2N), and extracted with chloroform/isopropanol (3:1). The pooled organic layers were dried over magnesium sulfate, and filtered. The filtrate was added silica gel, and the solvents were removed under vacuum. Purification by chromatography on silica gel (3% NH3 MeOH in dichloromethane) gave 4-(1-(1H-imidazol-4-yl)ethyl)-2,3-dimethylpyridine (H) as a white solid.

¹H NMR (300 MHz, CDCl₃) δ 8.14 (d, J = 5.1 Hz, 1H), 7.46 (s, 1H), 6.88 (d, J = 5.4 Hz, 1H), 6.72 (s, 1H), 4.35 (q, J = 6.9 Hz, 1H), 2.47 (s, 3H), 2.22 (s, 3H), 1.55 (d, J = 6.9 Hz, 3H).

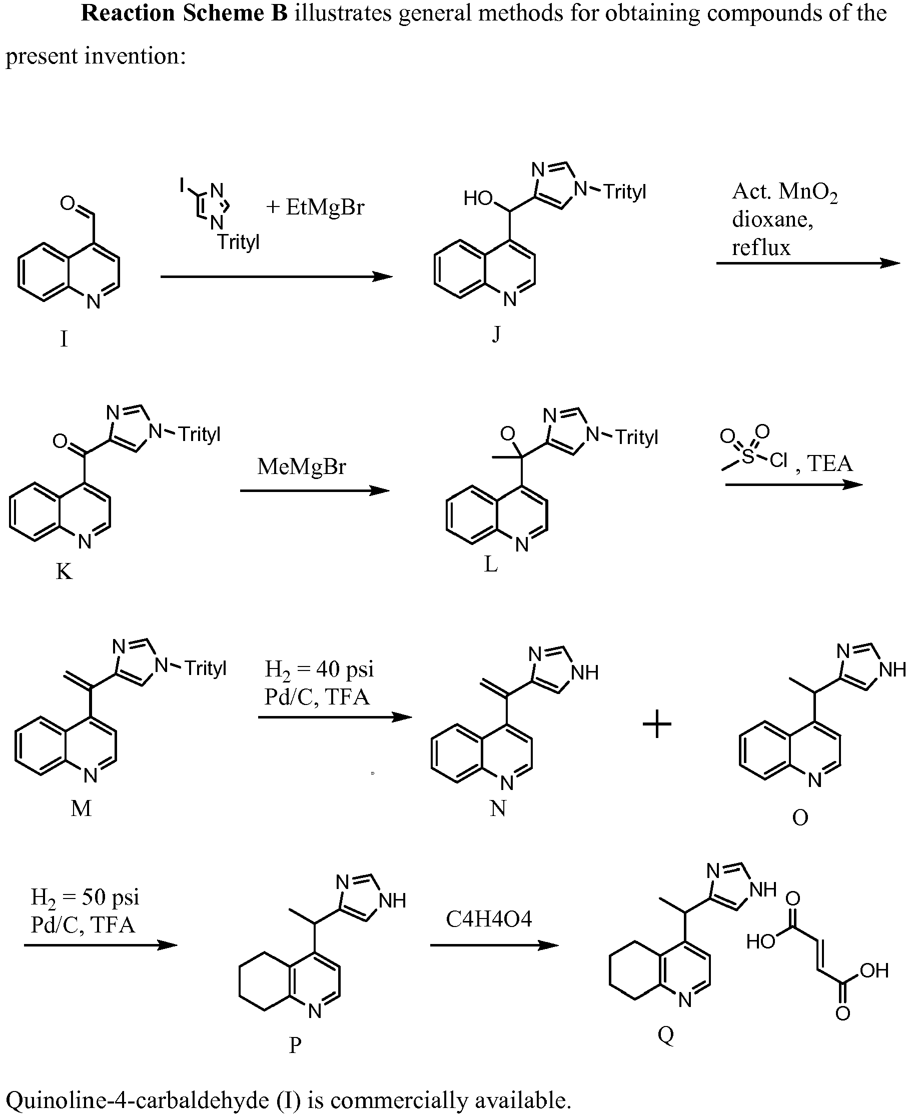

Quinolin-4-yl(1-trityl-1H-imidazol-4-yl)methanol (J): Use of I in the method to synthesize D to produce quinolin-4-yl(1-trityl-1H-imidazol-4-yl)methanol (J).

Quinolin-4-yl(1-trityl-1H-imidazol-4-yl)methanone (K): A mixture of J (6.14 g, 0.0131 mol) and activated manganese oxide (85%, 13.4 g, 0.131 mol) in dioxane (100 ml) was refluxed for 5 hours. The reaction mixture was cooled to room temperature, and filtered through a bed of celite. The solvent was removed under vacuum to give quinolin-4-yl(1-trityl-1H-imidazol-4-yl)methanone (K) as a white solid.

4-(1-(1-trityl-1H-imidazol-4-yl)vinyl)quinoline (M): Use of K in the methods to synthesize F, and G respectively to produce 4-(1-(1-trityl-1H-imidazol-4-yl)vinyl)quinoline (M).

4-(1-(1H-imidazol-4-yl)vinyl)quinoline (N): M (3.30 g, 0.00710 mol) in TFA was added palladium (10%, 0.48 g). The mixture was hydrogenated at 40 psi over night. The reaction mixture was filtered through a bed of celite to remove palladium catalyst. TFA was removed, and the residue was basified with ammonia in methanol (7 N). Solvent was removed. The residue was dissolved in ethyl acetate, and added silica gel. Solvent was removed. Purification by chromatography on silica gel (2-3% NH3 MeOH in dichloromethane) gave 4-(1-(1H-imidazol-4-yl)vinyl)quinoline (N), and 4-(1-(1H-imidazol-4-yl)ethyl)quinoline (O).

4-(1-(1H-imidazol-4-yl)ethyl)-5,6,7,8-tetrahydroquinoline (P): Use ofN in the method to synthesize N except hydrogenation was done at 50 psi to produce 4-(1-(1H-imidazol-4-yl)ethyl)-5,6,7,8-tetrahydroquinoline (P) as a clear oil.

4-(1-(1H-imidazol-4-yl)ethyl)-5,6,7,8-tetrahydroquinoline fumarate (Q): A solution of P (0.260 g, 0.00114 mol) in THF was added a solution of fumaric acid (0.130 g, 0.00111 mol) in ethanol. The resulting solution was removed under vacuum. The solid residue was recrystallized in THF/MeOH to give 4-(1-(1H-imidazol-4-yl)ethyl)-5,6,7,8-tetrahydroquinoline fumarate (Q) with the ratio of imidazole to fumaric acid of 1 to 1.13.

¹H NMR (300 MHz, DMSO-d⁶): δ 8.16 (d, J = 5.1 Hz, 1H), 7.61 (s, 1H), 6.91 (d, J = 5.1 Hz, 1H), 6.83 (s, 1H), 6.60 (s, 2.27H), 4.23 (q, J = 6.9 Hz, 1H), 2.82-2.74 (m, 4H), 1.84-1.70 (m, 4H), 1.43 (d, J = 6.9 Hz, 3H).

The results of the RSAT assay with several exemplary compounds of the invention are disclosed in Table 1 below together with the chemical formulas of these exemplary compounds. EC₅₀ values are nanomolar.

**TABLE I In Vitro Data**

| Structures | **RSAT** | | |
|---|---|---|---|
| | EC₅₀ (nM); efficacy | | |
| | Alpha 2A | Alpha 2B | Alpha 2C |
| 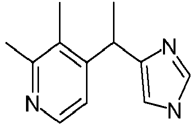 | Not active | 16.7 (0.99) | 1287 (0.35) |
| 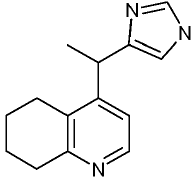 | Not active | 23.5 (1.12) | 370 (0.35) |

## Claims

1. A compound having the structure: 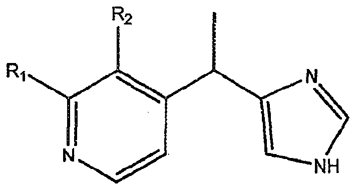 Wherein:
**R₁** and **R₂** independently= H, CH₂OR₃, C₁-C₄ alkyl, halogens, CF₃,
**R₃** = H, C₁ - C₄ alkyl
**R₁** and **R₂** may be fused to form a saturated ring
-CH₂-X-(CH₂)ₙ-CH₂-
X = CH₂,
n = 0,1,2
and any combination thereof or pharmaceutically acceptable salts, tautomers, enantiomers and diastereomers.

2. The compound of claim 1 wherein R₁ is CH₂OR₃ and R₂ is H.

3. The compound of claim 2 wherein R₃ is H.

4. The compound of claim 1 wherein R₁ CH₂OR₃ and R₂ is CH₃.

5. The compound of claim 1 wherein R₁ is H and R₂ is CH₂OH.

6. The compound of claim 1 wherein R₁ is CH₃ and R₂ is CH₂OH.

7. A compound according to claim 1 selected from the group consisting of: 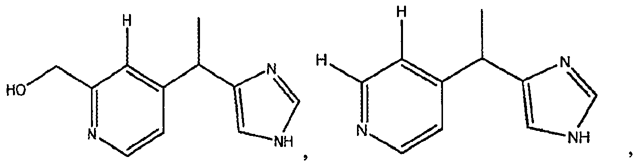 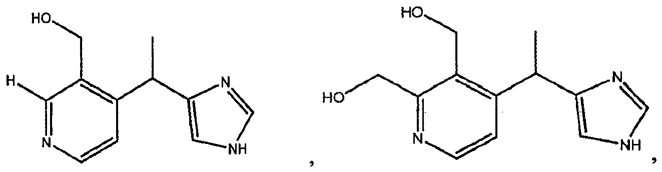 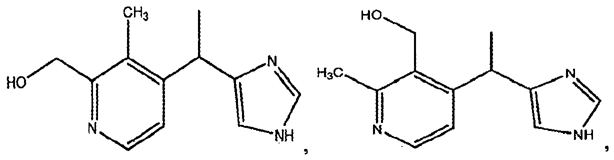 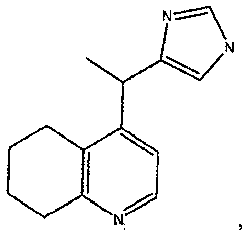 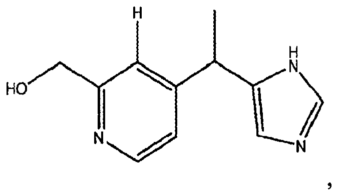 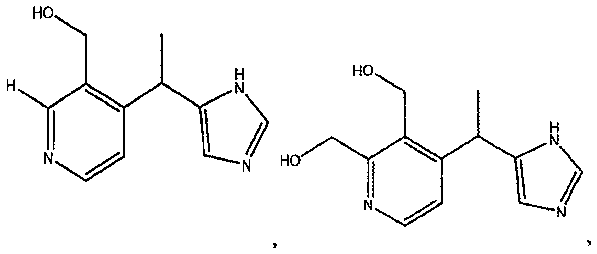 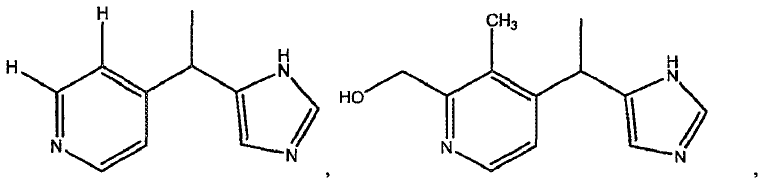 and

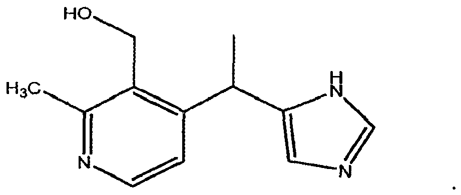

8. A compound according to claim 1 for use in a method of treating ischemic neuropathies, optic neuropathy, pain, visceral pain, corneal pain, headache pain, migraine, cancer pain, back pain, irritable bowel syndrome pain, muscle pain and pain associated with diabetic neuropathy, diabetic retinopathy, other retinal degenerative conditions, stroke, cognitive deficits, neuropsychiatric conditions, drug dependence and addiction, withdrawal symptoms, obsessive-compulsive disorders, obesity, insulin resistance, stress-related conditions, diarrhea, diuresis, nasal congestion, spasticity, attention deficit disorder, psychoses, anxiety, depression, autoimmune disease, Crohn's disease, gastritis, Alzheimer's, Parkinson's ALS, and other neurodegenerative diseases in a mammal by administering the compound to said mammal.

9. A compound according to claim 1 for use in a method of treating pain in a mammal by administering said compound to said mammal.

10. Compound for use according to claim 8 or 9, wherein the compound binds to the alpha 2B or alpha 2C receptor.

11. Compound for use according to claim 8 wherein the pain is chronic pain.

12. Compound for use according to claim 11 wherein the chronic pain is neuropathic pain.

13. Compound for use according to claim 11 wherein the chronic pain is associated with fibromyalgia.

14. Compound for use according to claim 9 wherein the pain is visceral pain.

## Patentansprüche

1. Verbindung mit der Struktur: 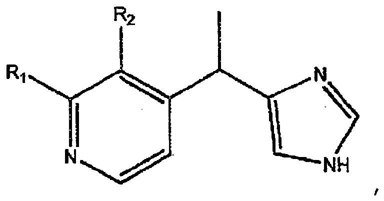 worin:
R₁ und R₂ unabhängig = H, CH₂OR₃, C₁₋₄-Alkyl, Halogen, CF₃;
R₃ = H, C₁₋₄-Alkyl;
R₁ und R₂ kondensiert sein können, um einen gesättigten Ring -CH₂-X-(CH₂)ₙ-CH₂ zu bilden;
X = CH₂;
n = 0, 1, 2
und irgendeine Kombination davon oder pharmazeutisch annehmbare Salze, Tautomere, Enantiomere und Diastereomere.

2. Verbindung gemäss Anspruch 1, worin R₁ CH₂OR₃ ist und R₂ H ist.

3. Verbindung gemäss Anspruch 2, worin R₃ H ist.

4. Verbindung gemäss Anspruch 1, worin R₁ CH₂OR₃ ist und R₂ CH₃ ist.

5. Verbindung gemäss Anspruch 1, worin R₁ H ist und R₂ CH₂OH ist.

6. Verbindung gemäss Anspruch 1, worin R₁ CH₃ ist und R₂ CH₂OH ist.

7. Verbindung gemäss Anspruch 1, ausgewählt aus der Gruppe bestehend aus: 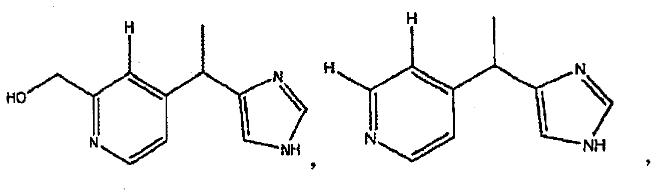 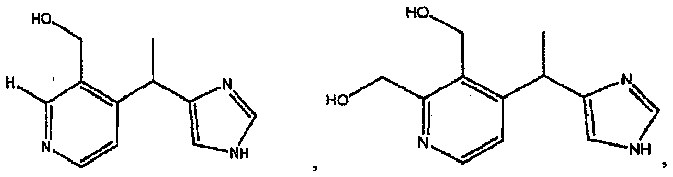 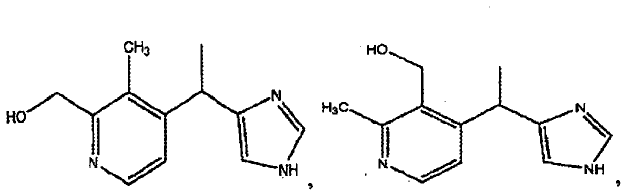 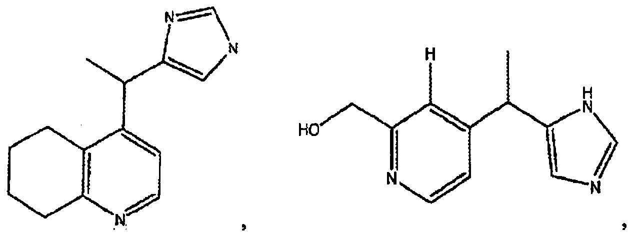 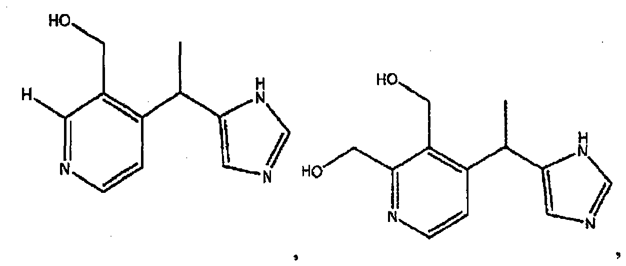 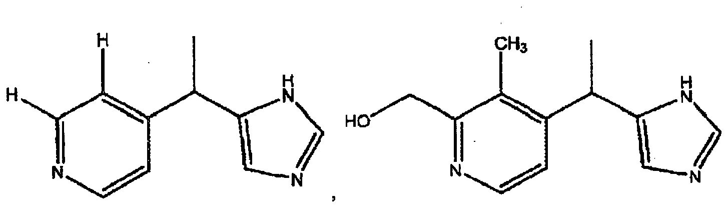 und

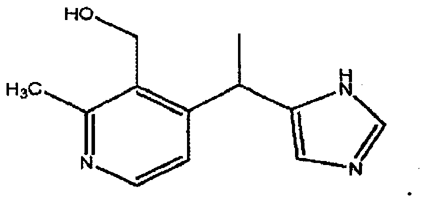

8. Verbindung gemäss Anspruch 1 zur Verwendung in einem Verfahren zur Behandlung von ischämischen Neuropathien, optischer Neuropathie, Schmerzen, viszeralen Schmerzen, Hornhautschmerzen, Kopfschmerzen, Migräne, Krebsschmerzen, Rückenschmerzen, Reizdarmsyndromschmerzen, Muskelschmerzen und Schmerzen, die mit diabetischer Neuropathie verbunden sind, diabetischer Retinopathie, anderen degenerativen Netzhautzuständen, Schlaganfall, kognitiven Defiziten, neuropsychiatrischen Zuständen, Drogenabhängigkeit und -sucht, Entzugserscheinungen, Zwangsstörungen, Fettleibigkeit, Insulinresistenz, stressbedingten Zuständen, Durchfall, Diurese, verstopfter Nase, Spastik, Aufmerksamkeits-Defizit-Störung, Psychosen, Angst, Depression, Autoimmunkrankheit, Morbus Crohn, Gastritis, Alzheimer, Parkinson-ALS und anderen neurodegenerativen Krankheiten in einem Säuger durch Verabreichung der Verbindung an diesen Säuger.

9. Verbindung gemäss Anspruch 1 zur Verwendung in einem Verfahren zur Behandlung von Schmerzen bei einem Säuger durch Verabreichen der Verbindung an diesen Säuger.

10. Verbindung zur Verwendung gemäss Anspruch 8 oder 9, wobei die Verbindung an den α-2B- oder α-2C-Rezeptor bindet.

11. Verbindung zur Verwendung gemäss Anspruch 8, wobei der Schmerz chronischer Schmerz ist.

12. Verbindung zur Verwendung gemäss Anspruch 11, wobei der chronische Schmerz ein neuropathischer Schmerz ist.

13. Verbindung zur Verwendung gemäss Anspruch 11, wobei der chronische Schmerz mit Fibromyalgie verbunden ist.

14. Verbindung zur Verwendung gemäss Anspruch 9, wobei der Schmerz ein viszeraler Schmerz ist.

## Revendications

1. Composé ayant la structure suivante : 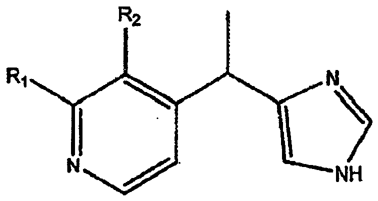 dans laquelle :
R₁ et R₂ indépendamment = H, CH₂OR₃, un groupe alkyle en C₁-C₄, des atomes d'halogène, CF₃,
R₃ = H, un groupe alkyle en C₁-C₄
R₁ et R₂ peuvent être fusionnés pour former un cycle saturé
-CH₂-X-(CH₂)ₙ-CH₂-
X = CH₂,
n = 0,1,2
et toute combinaison de ceux-ci ou des sels, tautomères, énantiomères et diastéréomères pharmaceutiquement acceptables.

2. Composé selon la revendication 1, dans lequel R₁ est CH₂OR₃ et R₂ est H.

3. Composé selon la revendication 2, dans lequel R₃ est H.

4. Composé selon la revendication 1, dans lequel R₁ est CH₂OR₃ et R₂ est CH₃.

5. Composé selon la revendication 1, dans lequel R₁ est H et R₂ est CH₂OH.

6. Composé selon la revendication 1, dans lequel R₁ est CH₃ et R₂ est CH₂OH.

7. Composé selon la revendication 1, sélectionné dans le groupe constitué de : 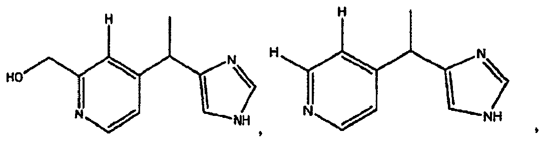 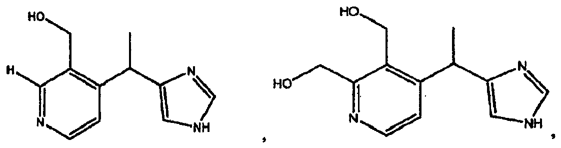 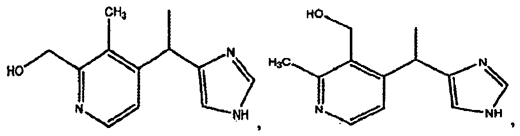 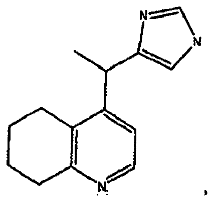 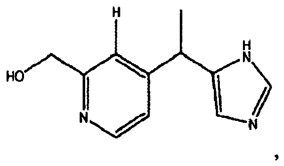 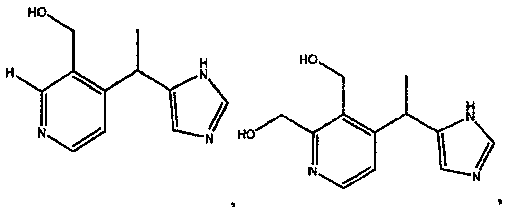 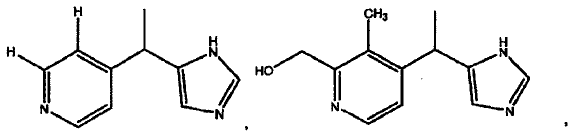 et

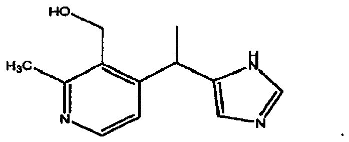

8. Composé selon la revendication 1, pour son utilisation dans un procédé de traitement de neuropathies ischémiques, d'une neuropathie optique, de la douleur, d'une douleur viscérale, d'une douleur cornéenne, d'une douleur liée aux céphalées, de la migraine, d'une douleur cancéreuse, d'une dorsalgie, d'une douleur liée au syndrome du côlon irritable, d'une douleur musculaire et d'une douleur associée à la neuropathie diabétique, de la rétinopathie diabétique, d'autres troubles de dégénérescence rétinienne, d'un accident vasculaire cérébral, de déficits cognitifs, de troubles neuropsychiatriques, de la toxicomanie et de l'accoutumance, des symptômes de sevrage, des troubles obsessionnels compulsifs, de l'obésité, de l'insulinorésistance, des affections liées au stress, de la diarrhée, de la diurèse, de la congestion nasale, de la spasticité, d'un trouble déficitaire de l'attention, des psychoses, de l'anxiété, de la dépression, d'une maladie auto-immune, de la maladie de Crohn, d'une gastrite, de la maladie d'Alzheimer, de la SLA de Parkinson, et d'autres maladies neurodégénératives
chez un mammifère par administration du composé audit mammifère.

9. Composé selon la revendication 1, pour son utilisation dans un procédé de traitement de la douleur chez un mammifère par administration dudit composé audit animal.

10. Composé pour son utilisation selon la revendication 8 ou 9, dans lequel le composé se lie au récepteur alpha 2B ou alpa 2C.

11. Composé pour son utilisation selon la revendication 8, dans lequel la douleur est une douleur chronique.

12. Composé pour son utilisation selon la revendication 11, dans lequel la douleur chronique est une douleur neuropathique.

13. Composé pour son utilisation selon la revendication 11, dans lequel la douleur chronique est associée à la fibromyalgie.

14. Composé pour son utilisation selon la revendication 9, dans lequel la douleur est une douleur viscérale.
